(19) Europäisches Patentamt
European Patent Office
Office européen des brevets

(11) Veröffentlichungsnummer: **0 088 963**
**A1**

# (12) EUROPÄISCHE PATENTANMELDUNG

(21) Anmeldenummer: 83102129.0

(22) Anmeldetag: 04.03.83

(51) Int. Cl.³: C 07 D 231/12

(30) Priorität: 13.03.82 DE 3209148

(43) Veröffentlichungstag der Anmeldung: 21.09.83 Patentblatt 83/38

(84) Benannte Vertragsstaaten: AT BE CH DE FR GB IT LI

(71) Anmelder: BASF Aktiengesellschaft
Carl-Bosch-Strasse 38
D-6700 Ludwigshafen(DE)

(72) Erfinder: Goetz, Norbert, Dr.
Schoefferstrasse 25
D-6520 Worms(DE)

(72) Erfinder: Rohr, Wolfgang, Dr.
In der Dreispitz 13
D-6706 Wachenheim(DE)

(72) Erfinder: Mangold, Dietrich, Dr.
Hermann-Walker-Strasse 49
D-6903 Neckargemuend(DE)

(72) Erfinder: Hupfer, Leopold, Dr.
Waltershoehe 3
D-6701 Friedelsheim(DE)

(72) Erfinder: Dockner, Toni, Dr.
Grossgasse 6
D-6701 Meckenheim(DE)

(72) Erfinder: Kempe, Uwe, Dr.
Danziger Strasse 9
D-6701 Dannstadt-Schauernheim(DE)

(54) **Verfahren zur Herstellung von Pyrazolen durch katalytische Dehydrierung von Pyrazolinen.**

(57) Pyrazole der Formel I

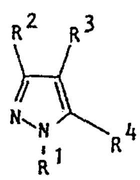

(I)

in der

$R^1$ bis $R^4$ gleiche oder verschiedene Substituenten bedeuten, die Wasserstoff-, Alkyl-, Aralkyl- oder Arylreste sein können, erhält man durch Dehydrierung aus entsprechenden Pyrazolinen in der Gasphase bei einer Temperatur von 150 bis 400°C an einem Palladium oder Platinkatalysator.

EP 0 088 963 A1

0088963

BASF Aktiengesellschaft

O.Z. 0050/35791

# Verfahren zur Herstellung von Pyrazolen durch katalytische Dehydrierung von Pyrazolinen

Die Erfindung betrifft ein neues Verfahren zur Herstellung von Pyrazolen der allgemeinen Formel I

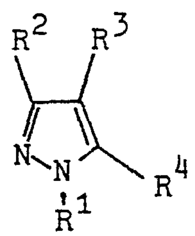

(I)

in der

$R^1$ bis $R^4$ gleich oder verschieden sein können und jeweils ein Wasserstoffatom, aliphatische, araliphatische oder aromatische Reste mit 1 bis 10 Kohlenstoffatomen bedeuten, durch Dehydrierung von Pyrazolinen.

Aus Chemistry of Carbon Compounds, Band IV a (Elsevier, N.Y., 1957), Seiten 246 bis 249, sind zahlreiche Synthesen von Pyrazolen bekannt, z.B. durch Umsetzung von Hydrazinen mit 1,3-Dicarbonylverbindungen, Dehydratation von Hydrazonen aus ß-Ketoestern, Cyclisierung von Hydrazonen aus ß-Cyanoketonen, Umsetzung von Aldehydphenylhydrazonen mit ß-Ketoestern in Gegenwart von Zinkchlorid oder durch Reaktion von Diazoverbindungen mit Acetylenverbindungen. Die bedeutenderen Synthesewege führen über die Umsetzung von Derivaten von 1,3-Dicarbonylverbindungen mit Hydrazin oder Hydrazinverbindungen in wäßriger, saurer Lösung oder mit Salzen des Hydrazins oder von Hydrazinderivaten zu verdünnten wäßrigen Lösungen von Pyrazolen.

Mu/P

Nachteilig ist bei allen diesen Synthesewegen, daß - insbesondere im Fall des unsubstituierten Pyrazols oder der in 1- bzw. 4-Stellung substituierten Pyrazole - auf schwierig zugängliche und aufwendig herzustellende Zwischenprodukte zurückgegriffen werden muß. Vorteilhafter ist es, Pyrazol bzw. substituierte Pyrazole durch Dehydrierung des technisch gut zugänglichen Pyrazolins bzw. substituierten Pyrazolins herzustellen.

Für die Herstellung von Pyrazolin und substituierten Pyrazolinen vgl. Curtius, Wirsing, J. prakt. Chemie (2) 50, S. 531 bis 554 (1894).

Als Dehydrierungsmethode ist bisher die Umsetzung von Pyrazolinen mit elementarem Schwefel (oder Selen) zu Pyrazolen beschrieben (Grandberg und Kost, J. Gen. Chem. 28, 3102 (1958)). Als Nachteil ist bei diesem Verfahren die zusätzliche Bildung von Schwefelwasserstoff anzusehen, der entweder beseitigt oder durch Oxidation wieder in Schwefel überführt werden muß. Erschwerend kommen noch die Geruchsbelästigung und Giftigkeit von Schwefelwasserstoff hinzu.

Bekannt ist die Dehydrierung von hydrierten aromatischen Systemen zu Aromaten bzw. Heteroaromaten an Edelmetallkatalysatoren (Houben-Weyl, Methoden der Org. Chemie", Band 4/2, S. 338 ff; N. D. Zelinsky, J. K. Jurjew, Chem. Ber. 64 (1931), S. 101), wobei diese Reaktion allerdings nur für Verbindungen wie Cyclohexan, substituierte Cyclohexane oder Pyrrolidin beschrieben wird, die gegenüber Wasserstoff bei erhöhter Temperatur stabil sind. Beispielsweise ist die Dehydrierung von N-Methylpyrrolidin gut durchführbar (US-PS 3 522 269, US-PS 3 008 965, GB-PS 1 393 086). Anders sieht es bei den Pyrazolinen aus, die mit ihrer N-N-Bindung eine gegenüber hydrogenolytischen Angriffen sehr empfindliche Stellen besitzen, wie man

leicht anhand der Hydrierung von Hydrazonen erkennen kann (F. Zymalkowski "Katalytische Hydrierungen" Verlag Ferd. Enke (1965), S. 304 bis 309; Rylander "Catalytic Hydrogenation over Platinum Metals" Academic Press (1967), S. 134 bis 138; Freifelder "Practical Catalytic Hydrogenation" John Wiley a. Sons (1971), S. 320 bis 322). Solche Verbindungen werden in Gegenwart von Wasserstoff leicht unter Spaltung der N,N-Bindung in die entsprechenden Amine überführt. Da bei Dehydrierungen immer Wasserstoff entsteht und in manchen Fällen - zur Erhaltung der Aktivität des Katalysators - auch Wasserstoff zugegen sein muß, sind hierbei ebenfalls Spaltreaktionen zu erwarten.

Es wurde nun gefunden, daß man Pyrazole der eingangs näher bezeichneten Formel I in guter Ausbeute erhält, wenn man auf ein Pyrazolin der allgemeinen Formel II

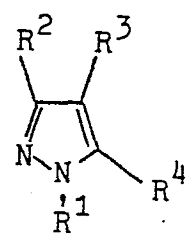

in der $R^1$ bis $R^4$ die oben angegebene Bedeutung haben, bei einer Temperatur zwischen 150 und 400°C, vorzugsweise zwischen etwa 200 und 300°C in der Gasphase einen Palladium- oder Platinkatalysator einwirken läßt.

Ausgangsstoffe II können beispielsweise sein: Pyrazolin, 3-Methyl-, 5-Methylpyrazolin, 3-Ethylpyrazolin, 3-Propylpyrazolin, 1-Phenylpyrazolin, 1,5-Dimethylpyrazolin, 3,5-Dimethylpyrazolin, 1-Phenyl-3-methyl-pyrazolin, 3,4,5-Trimethylpyrazolin.

Die Umsetzung wird in der Gasphase bei ggf. erhöhtem oder vermindertem Druck durchgeführt. Die Pyrazoline II können lösungsmittelfrei oder in einem Lösungsmittel einer auf entsprechende Temperatur gebrachten Reaktionszone zugeführt werden, die den Katalysator enthält. Die zunächst flüssigen Reaktionsteilnehmer kann man entweder verdampfen und einem Trägergasstrom beimischen oder unmittelbar auf dem Katalysator verdampfen.

Als Lösungsmittel kommen beispielsweise aromatische Kohlenwasserstoffe, z.B. Toluol, Ethylbenzol, o-, m- und p-Xylol, Isopropylbenzol; Alkanole und Cycloalkanole wie Methanol, Ethanol, Propanol, Isopropanol, n-Butanol, Cyclohexanol, Ethylenglykolmonomethylether, 2-Ethyl-hexanol, Methylcyclohexanol; Ether, z.B. Di-n-butylether, Cyclohexylmethylether, Anisol, Resorcindimethylether, Ethylenglykoldimethylether, Tetrahydrofuran, Dioxan, tertiäre Amine, z.B. Triethylamin, Triisobutylamin, N,N-Dimethylisopropylamin, N-Methylpiperidin, N-Methylmorpholin in Frage. Zweckmäßig verwendet man das Lösungsmittel in einer Menge von 0,5- bis 10-fach , bezogen auf den Ausgangsstoff II. Die Dehydrierungsprodukte selbst können das Lösungsmittel ganz oder teilweise in Fällen bilden, wo die Umsetzung wiederholt wird, um sie zu vervollständigen.

Als Trägergas wird, falls gewünscht, vorzugsweise Stickstoff oder Ammoniak verwendet und soviel Wasserstoff, daß der Katalysator aktiv bleibt.

Das nach dem Verfahren der Erfindung verwendete Katalysatorsystem enthält Palladium oder Platin, zweckmäßig auf einem Träger. Die Metalle können auf dem Träger allein verwendet werden, günstig ist aber die Verwendung gewisser Zusätze, nämlich Elementen bzw. Verbindungen von Elementen der Gruppe 1b, 2b und 7b des Periodensystems sowie Eisen,

Kobalt und Nickel, unabhängig hiervon die Verwendung basischer Stoffe, d.h. Verbindungen der ersten bis dritten Hauptgruppe des Periodensystems. Die letzteren, basischen Stoffe bilden vorzugsweise einen Bestandteil des Trägers. Die Zusätze können allein oder im Gemisch miteinander neben dem Edelmetall vorliegen. Die aktiven Bestandteile des Katalysators sind auf einem Träger aufgebracht, der aus Aluminiumoxid oder einem unter Mitwirkung von Aluminiumoxid aufgebauten Stoff besteht. Aluminiumsilikat oder Spinelle des Aluminiums sind solche Stoffe. Die Zusätze, die dem Katalysator seine Eigenschaften verleihen, können sich an der Oberfläche des Katalysators befinden, zusammen mit dem übrigen Aluminiumoxid u.a. in Form einer Mischung den Träger darstellen oder mit ursprünglichen Träger durch nachträgliches Tempern ein gemeinsames Kristallgitter bilden (beispielsweise bei Aluminiumoxidträgern Bildung von typischen Spinellgittern mit bestimmten Metallen). Das bedeutet, daß die aktiven Zusätze bzw. die entsprechend hergestellten Träger Aktivität und auch Lebensdauer des Katalysators günstig beeinflussen können.

Als Zusätze kommen in Frage:

a) Basische Zusätze, wie Oxide, Hydroxide oder Carbonate der Alkalimetalle, vorzugsweise des Lithiums und Natriums, der Erdalkalimetalle, vorzugsweise des Magnesiums und Calciums und der Seltenerdmetalle, vorzugsweise des Cers und Praseodyms Neodyms.

b) Weitere Metalle, wie Eisen, Nickel, Kobalt, Mangan, Zink, Cadmium, Silber.

Die Zusätze können gemeinsam mit dem Edelmetall durch Tränken des Trägermaterials mit Lösungen von z.B. Nitraten, Chloriden, Formiaten oder Oxalaten aufgebracht werden. Durch anschließende Behandlung, die gewöhnlich zwischen 400 und 600°C durchgeführt wird, werden die aufgebrachten Verbindungen gewöhnlich in die Oxide überführt. Soll bei Aluminiumoxidträgern mit geeigneten Metallen (Mg, Zn, Co, Mn, Li) Spinellbildung erzielt werden, muß nach der Tränkung auf eine Temperatur von 900 bis 1300°C erhitzt werden (siehe Ullmanns Encyklopädie der technischen Chemie, 3. Auflage (1955) Band 6, 242 bis 244, Gmelin, System-Nr. 35, Al, Tl, 1934 bis 1935, S. 26 bis 28) und anschließend in der üblichen Weise das Edelmetall aufgebracht werden.

Manche Zusätze, wie beispielsweise Calciumoxid oder Magnesiumoxid lassen sich mit einem vorgeformten Träger wie Aluminiumoxid mischen und bilden dann nach gemeinsamem Tempern bei 400 bis 600°C einen neuen Träger, auf dem das Edelmetall niedergeschlagen werden kann.

Der Edelmetallgehalt des Katalysators, bezogen auf das Trägermaterial, liegt gewöhnlich zwischen 0,05 bis 15 Gew.%.

Das Gewichtsverhältnis der Zusätze zum Edelmetall kann unterschiedlich sein; es kann z.B. zwischen 10 000 : 1 bis 1 : 50, bevorzugt bei 100 : 1 bis 1 : 50 liegen. Man verwendet den Katalysator beispielsweise in Form von Strängen mit einem Durchmesser von 3 mm und einer Länge von 10 mm oder als Pulver, je nach dem vorgesehenen Anwendungszweck.

Pyrazol und substituierte Pyrazole werden z.B. zur Herstellung von Pflanzenschutzmitteln und Pharmazeutika verwendet.

Herstellungsmöglichkeiten für den Katalysator

a) Auf strang- oder pulverförmiges gamma-Aluminiumoxid wird das Edelmetall zusammen mit dem Zusatz (Mangan, Zink, Silber, Seltenerdmetalle u.a.) in der gewünschten Menge durch Tränken mit beispielsweise deren Nitratlösungen und anschließendes Eindampfen bis zur Trockene aufgebracht. Danach wird 6 Stunden bei 550°C getempert und im Wasserstoffstrom bei 300°C reduziert.

b) Auf strang- oder pulverförmiges gamma-Aluminiumoxid wird zunächst die gewünschte Menge des Zusatzes (Mangan, Zink, Cobalt, Magnesium, Lithium u.a.) durch Tränken mit entsprechenden wäßrigen Nitrat- oder Formiatlösungen aufgebracht und bei 150°C getrocknet. Der derart vorbehandelte Träger wird nun entweder 6 Stunden lang auf 550°C erhitzt oder, wenn Spinellbildung erreicht werden soll, 6 Stunden lang bei 1050°C geglüht. Nun wird der Träger mit wäßriger Edelmetallnitratlösung getränkt und durch 7stündiges Erhitzen bei 300°C im Wasserstoffstrom reduziert. Wurde zum Tränken eine Lösung eines Chlorids genommen, wird mit alkalischer Formalinlösung reduziert.

c) gamma-Aluminiumoxid und ein basisches Oxid (MgO, CaO) werden in dem gewünschten Mengenverhältnis intensiv miteinander vermischt. Diese Mischung wird 6 Stunden lang auf 450°C erhitzt, anschließend mit Edelmetallnitratlösung getränkt und 7 Stunden mit Wasserstoff bei 300°C reduziert. Der Katalysator wird mit einer 5 %igen wäßrigen Hydrazinhydratlösung nachreduziert und dann bei 120°C getrocknet.

Die in den nachfolgenden Beispielen angegebenen Siedepunkte beziehen sich, sofern nicht anders angegeben,

auf atmosphärischen, sonst auf verminderten Druck in mbar.

Beispiel 1

In ein zylindrisches Reaktionsrohr mit einem Volumen von 1 l wird ein Katalysator in Form von Strängen (4 mm Durchmesser, 10 mm Länge), der 0,5 Gew.% Palladium auf einer Mischung aus 19,4 Gew.% Magnesiumoxid und 80,6 Gew.% Aluminiumoxid enthält, eingefüllt und auf 250°C erhitzt. Über dieses Katalysatorbett werden stündlich 50 g Pyrazolin, die in 50 ml N-Methylmorpholin gelöst und in einer Mischung aus 400 Nl Stickstoff und 10 Nl Wasserstoff (pro Stunde) verdampft sind, hindurchgeleitet. Das Reaktionsprodukt wird, sobald es den Reaktor verläßt, abgekühlt und durch Destillation gereinigt.

Aus 100 g Pyrazolin erhielt man bei dieser Arbeitsweise 74 g Pyrazol (Kp = 186 bis 187°C, Fp = 69 - 70°C), entsprechend einer Ausbeute von 76 % d.Th.

Beispiel 2

Man verfährt analog Beispiel 1, verwendet aber einen Katalysator, der 1,8 Gew.% Palladium auf einem Lithium-Aluminiumspinell enthält, bei einer Reaktionstemperatur von 210°C.

Man erhielt aus 3-Methylpyrazolin das entsprechende 3-Methylpyrazol (Kp = 204 bis 206°C) in einer Ausbeute von 84 % d.Th.

Beispiel 3

Man verfährt analog Beispiel 1, verwendet aber einen Katalysator, der 1,0 Gew.% Palladium auf Cobalt-Aluminiumspinell enthält. Bei einer Reaktionstemperatur von 230°C wurde aus 1,5-Dimethylpyrazolin das entsprechende 1,5-Dimethylpyrazol (Kp = 153°C) in einer Ausbeute von 87 % d.Th. erhalten.

Beispiel 4

Man verfährt analog Beispiel 1, verwendet aber einen Katalysator, der 0,5 Gew.% Palladium auf Magnesium-Aluminiumspinell enthält; man erhielt aus 1-Phenylpyrazolin das entsprechende 1-Phenylpyrazol (Kp = 246 bis 247°C) in einer Ausbeute von 93 % d.Th.

Beispiel 5

In einen Wirbelbettreaktor mit einem Fassungsvermögen von 1,3 l wird 1 l Katalysator eingefüllt. Der Katalysator hat die Zusammensetzung 0,5 Gew.% Palladium, 0,11 Gew.% Zink und 0,10 Gew.% Cadmium auf Aluminiumoxid und besitzt eine Korngröße von 0,2 bis 0,6 mm. Die Temperatur des Reaktors wird auf 230°C eingestellt und eine entsprechend vorerhitzte Mischung aus 10 Nl Wasserstoff und 400 Nl Stickstoff pro Stunde eingeleitet. Der so erzeugten Katalysator-Wirbelschicht werden stündlich 100 g einer Lösung von 50 g Pyrazolin in 50 ml N-Methylpiperidin zugeführt. Das durch Abkühlung gewonnene Reaktionsprodukt wird durch Destillation aufgearbeitet. Bei dieser Arbeitsweise konnten aus 100 g Pyrazolin 71 g Pyrazol, entsprechend einer Ausbeute von 73 % d.Th. erhalten werden.

Beispiel 6

Man verfährt analog Beispiel 5, verwendet aber einen Katalysator, der 0,5 Gew.% Palladium, 5,0 Gew.% Silber und 1,0 Gew.% Mangan auf Aluminiumoxid enthält; aus 3,5-Dimethylpyrazolin wurde das entsprechende 3,5-Dimethylpyrazol (Kp = 218 bis 219°C) in einer Ausbeute von 82 % d.Th. erhalten.

Beispiel 7

Man verfährt analog Beispiel 5, verwendet aber einen Katalysator, der 0,5 Gew.% Palladium, 5,0 Gew.% Praseodymoxid und 1,0 Gew.% Mangan auf Aluminiumoxid enthält; aus 1-Phenyl-5-methyl-pyrazolin wurde das entsprechende 1-Phenyl-5-methyl-pyrazol (Kp = 128 bis 129/13,5 mbar) in einer Ausbeute von 95 % d.Th. erhalten.

Beispiel 8

In einen Rohrreaktor mit einem Fassungsvermögen von 1 l wird ein Katalysator in Form von Strängen (3 mm Durchmesser, 10 mm Länge), der 0,5 Gew.% Palladium auf 19,4 Gew.% Calciumoxid und 80,6 Gew.% Aluminiumoxid enthält, eingefüllt und auf 250°C erhitzt. Über dieses Katalysatorbett werden stündlich 100 ml Pyrazolin und eine Mischung aus 350 Nl gasförmigem Ammoniak und 10 Nl Wasserstoff pro Stunde im Gleichstrom hindurchgeleitet. Das Reaktionsprodukt wird - sobald es den Reaktor verläßt, abgekühlt und destillativ aufgearbeitet.

Man erhielt aus 100 g eingesetztem Pyrazolin 69 g Pyrazol, entsprechend einer Ausbeute von 71 % d.Th.

Beispiel 9

Man verfährt analog Beispiel 8, verwendet aber einen Katalysator, der 0,5 Gew.% Platin auf Aluminiumoxid enthält; es wurde, ausgehend von 4-Methylpyrazolin, das entsprechende 4-Methylpyrazol (Kp = 205 bis 206°C) in einer Ausbeute von 81 % d.Th. erhalten.

Beispiel 10

Aus einem beheizten Dosiergefäß wurden stündlich 28 g Pyrazolin in einen auf 200°C erhitzten Verdampfer und von dort zusammen mit 645 Nl Stickstoff durch einen auf 205 bis 210°C geheizten Rohrreaktor geleitet, der 0,2 l eines Katalysators in Strangform [Durchmesser 4 mm; 0,25 Gew.% Pd auf gamma-Aluminiumoxid] enthielt. Bei einem Umsatz von 90 % erhielt man jeweils 16,15 g Pyrazol; dies entspricht einer Selektivität von 66 %.

Patentansprüche

1. Verfahren zur Herstellung von Pyrazolen der Formel I

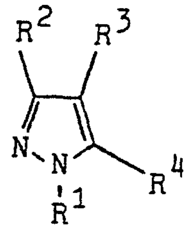

(I)

in der
$R^1$ bis $R^4$ gleiche oder verschiedene Substituenten bedeuten, die Wasserstoffatome, Alkyl-, Aralkyl- oder Arylreste sein können, durch Dehydrierung entsprechender Pyrazoline, dadurch gekennzeichnet, daß man auf die Pyrazoline in der Gasphase bei einer Temperatur von 150 bis 400°C einen Palladium- oder Platinkatalysator einwirken läßt.

2. Verfahren gemäß Anspruch 1, dadurch gekennzeichnet, daß man einen Katalysator verwendet, der einen basischen Stoff und/oder ein Element aus der Gruppe 1b, 2b oder 7b des Periodensystems, Eisen, Cobalt oder Nickel enthält.

3. Verfahren gemäß Anspruch 2, dadurch gekennzeichnet, daß der Katalysator als basischen Stoff ein Oxid, Hydroxid oder Carbonat eines Alkalimetalls, Erdalkalimetalls oder Seltenerdmetalls enthält.

4. Verfahren gemäß Anspruch 1 bis 3, dadurch gekennzeichnet, daß man die Umsetzung in Anwesenheit eines tertiären Amins oder von Ammoniak und ggf. eines Trägergases vornimmt.

Europäisches Patentamt

# EUROPÄISCHER RECHERCHENBERICHT

0088963
Nummer der Anmeldung

EP 83 10 2129

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | US-A-3 952 010 (M. GARBER et al.)<br>* Patentschrift *<br>----- | 1 | C 07 D 231/12 |
| | | | RECHERCHIERTE SACHGEBIETE (Int. Cl. 3) |
| | | | C 07 D 231/00 |

Der vorliegende Recherchenbericht wurde für alle Patentansprüche erstellt.

| Recherchenort | Abschlußdatum der Recherche | Prüfer |
|---|---|---|
| DEN HAAG | 29-06-1983 | CREMERS K. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03.82